# EUROPEAN PATENT SPECIFICATION

(11) **EP 1 908 392 B1**
(45) Date of publication and mention of the grant of the patent: **14.04.2010**
(21) Application number: 07019371.9
(22) Date of filing: 02.10.2007
(51) Int. Cl.: A61B 1/015

(54) **Endoscope**
Endoskop
Endoscope

(30) Priority: 04.10.2006 JP 2006273360
(43) Date of publication of application: 09.04.2008
(73) Proprietor: OLYMPUS MEDICAL SYSTEMS CORP., Shibuya-ku Tokyo 151-0072 (JP)
(72) Inventor: Ichimura, Hironobu, Hachioji-shi Tokyo 192-8512 (JP)
(74) Representative: von Hellfeld, Axel

(56) References cited:
- EP-A- 1 692 995
- WO-A-2006/011488
- JP-A- 3 106 329
- JP-A- 6 154 155
- JP-A- 2003 220 018

## Description

The present invention relates to an endoscope provided with an observation optical system that observes an object in a state that a distal end section of an objective optical system comes in contact with or it comes close thereto.

JPN.PAT.APPLN.KOKAI PUBLICATION NO.2005-640 publication (Patent Document 1) discloses an endoscope provided with an observation optical system of an object-contacting type and an ordinary observation optical system. The observation optical system of an object-contacting type is configured to observe an object in such a state that a distal end section of an objective optical system is brought into contact with the object. The ordinary observation optical system is configured to observe an object in a non-contacting state of an object optical system thereof with the object. Here, in the endoscope described in Patent Document 1, a projecting section projecting forward is provided on a distal end face of an insertion section of the endoscope. The observation optical system of an object-contacting type is disposed on an end face of the projecting section. Further, an observation window for the ordinary observation optical system, a plurality of illumination windows for an illumination optical system, an air-supply/water-supply nozzle, a distal end opening section of an endo-therapy device insertion channel, and the like are provided on an end face on the projecting section of the insertion section on a proximal end side thereof.

In such a case that the insertion section of the endoscope is inserted into a lumen, the projecting section of the insertion section is kept in non-contacting state with an object. In this case, endoscope observation is conducted by the ordinary observation optical system. An object contacting observation where the observation optical system of an object-contacting type is brought into contact with an object and the object is observed is performed in a state that the projecting section of the insertion section is brought into contact with an object.

JPN.PAT.APPLN.KOKAI PUBLICATION NO.2003-220018 publication (Patent Document 2) discloses an endoscope with a configuration that a liquid-feeding pipe conduit is provided independently from a pipe conduit for supplying cleaning liquid to a cleaning nozzle for an observation window cleaning that cleans an observation window at an insertion section distal end. An object to be observed can be stained by jetting stain solution from the liquid-feeding pipe conduit.

When the object contacting observation where the observation optical system of an object-contacting type is brought into contact with an object such as body tissue and the object is observed is conducted like the apparatus shown in Patent Document 1, it is desirable that cell tissue is stained by stain solution before contacting observation.

The endoscope disclosed in Patent Document 2 is configured such that an opening section of a liquid-feeding pipe conduit is formed on a peripheral edge section of a hood or a cap attached to and detached from the insertion section distal end of the endoscope and cell tissue to be observed in a contacting manner is stained by jetting stain solution from the opening section of the liquid-feeding pipe conduit. In this case, however, since a position of the opening section of the liquid-feeding pipe conduit is spaced from a site where cell observation should be conducted, it is difficult to stain the site where cell observation should be conducted accurately. Therefore, after cell tissue to be observed in a contacting manner is cleaned before the object-contacting observation is conducted, the workability of the endoscope when contacting observation of body tissue is conducted may be reduced because of for a procedure such as repeatedly spraying stain solution.

Also describing endoscopes with a pipe conduit opening section for fluid supply supplying fluid to the distal end phase are documents EP 1 692 995 A1, JP 03-1063291, JP 06-154155 and WO 2006/011488 A1.

The present invention has been made under these circumstances, and an object thereof is to provide an endoscope which can correctly stain a site where cell observation should be conducted when contacting observation of body tissue is conducted or an object is observed in a proximity state thereto and can improve workability of contacting observation.

An endoscope according to one aspect of the present invention is an endoscope according to claim 1 that includes an observing section that observes a body to be examined in a contacting state with the body to be examined or a proximal state to the body to be examined on a distal end face of an insertion section to be inserted into the body to be examined, comprising a pipe conduit opening section for fluid supply that supplies fluid to the distal end face; and a flow passage section that guides fluid caused to flow from the pipe conduit opening section in a direction of an observation window of the observing section.

With the configuration, the distal end face of the insertion section to be inserted into a body to be examined is brought into contact with a body to be examined at a contacting observation time of the body to be examined. In this state, fluid such as stain solution caused to flow from the pipe conduit opening section for fluid supply is guided toward the observation window by the flow passage section.

The flow passage section is a guide groove formed in a recessed shape on the distal end face.

With the configuration, in a state that the distal end face of the insertion section to be inserted into a body to be examined has been brought into contact with a body to be examined at a contacting observation time of the body to be examined, fluid such as stain solution caused to flow from the pipe conduit opening section for fluid supply is guided toward the observation window by the guide groove formed on the distal end face in a recessed shape.

It is preferable that the flow passage section is a chamfered section provided on a ridge line of the pipe conduit opening section and a chamfer amount of the chamfered section is large only in a direction of the observation window.

With the configuration, in a state that the distal end face of the insertion section to be inserted into a body to be examined has been brought into contact with a body to be examined at a contacting observation time of the body to be examined, fluid such as stain solution caused to flow from the pipe conduit opening section for fluid supply is guided toward the observation window by the chamfered section which is provided on the ridge line of the pipe conduit opening section and whose chamfer amount is large only in the direction of the observation window.

It is preferable that the insertion section includes a pipe conduit for fluid supply communicating with the pipe conduit opening section and the pipe conduit is disposed approximately parallel to an axial direction of the center axis of the insertion section.

With the configuration, by disposing the pipe conduit for fluid supply approximately parallel to the axial direction of the center axis of the insertion section, a diameter of the insertion section is prevented from increasing.

It is preferable that the flow passage section includes a projecting section which is caused to project forward at a peripheral site of the pipe conduit opening section on the distale end face and forms a gap for guiding fluid caused to flow from the pipe conduit opening section toward the direction of the observation window between the peripheral site of the pipe conduit opening section and the body to be examined when the distal end face is brought into contact with the body to be examined.

With the configuration, in a state that the distal end face of the insertion section to be inserted into a body to be examined has been brought into contact with a body to be examined at a contacting observation time of the body to be examined, a gap is formed between the peripheral site of the pipe conduit opening section and the body to be examined by pushing the body to be examined by the projecting section of the flow passage section so that fluid caused to flow from the pipe conduit opening section is guided toward the observation window via the gap.

It is preferable that the flow passage section includes a guide groove formed in a recessed shape on the distal end face and a projecting section which is caused to project forward a peripheral site of the pipe conduit opening section on the distal end face and the projecting section is disposed at a peripheral site of the guide groove.

With the configuration, in a state that the distal end face of the insertion section has been brought into contact with a body to be examined at a contacting observation time of the body to be examined, a gap is formed between the peripheral site of the pipe conduit opening section and a body to be examined by pushing the body to be examined by the projecting section disposed at the peripheral site of the guide groove so that fluid such as stain solution is guided toward the observation window via the guide groove on the distal end face and the gap.

It is preferable that the projecting section includes a pair of projecting pieces disposed on both sides of the guide groove.

With the configuration, in a state that the distal end face of the insertion section has been brought into contact with a body to be examined at a contacting observation time of the body to be examined, a gap is formed between the pair of projecting pieces on both the sides of the guide groove and the body to be examined by pushing the body to be examined by the pair of projecting pieces disposed on both the sides of the guide groove so that fluid such as stain solution is guided toward the observation window via the guide groove on the distal end face and the gap.

It is preferable that the projecting section is a nozzle for jetting cleaning water for cleaning the observation window.

With the configuration, in a state that the distal end face of the insertion section to be inserted into a body to be examined has been brought into contact with a body to be examined at a contacting observation time of the body to be examined, a gap is formed between a peripheral edge site of the pipe conduit opening section and the body to be examined by pushing the body to be examined by the jetting nozzle for cleaning water so that fluid caused to flow from the pipe conduit opening section is guided toward the observation window via the gap.

It is preferable that the pipe conduit opening section is caused to communicate with stain solution supplying means for supplying stain solution staining the body to be examined.

With the configuration, stain solution staining a body to be examined is supplied from the stain solution supplying means caused to communicate with the pipe conduit opening section and stain solution caused flow from the pipe conduit opening section is guided toward the observation window by the flow passage section.

It is preferable that the pipe conduit opening section is connected to the stain solution supplying means for supplying stain solution staining the body to be examined and cleaning water supplying means for supplying cleaning water, respectively, and an operation section of the endoscope comprises control means for conducting selective switching between the stain solution supplying means and the cleaning water supplying means to drive the switched means.

With the configuration, by conducting selective switching between the stain solution supplying means and the cleaning water supplying means to drive the switched means by the control means of the operation section of the endoscope, selective switching is performed between a state that cleaning water is supplied from the cleaning water supplying means and a state that stain solution is supplied from the stain solution supplying means.

An endoscope according to another aspect of the present invention is an endoscope that includes an observing section that observes a body to be examined in a contacting state with the body to be examined or a proximal state with the body to be examined on a distal end face of an insertion section to be inserted into the body to be examined, wherein a pipe conduit opening section for fluid supply that supplies fluid to the distal end face and a flow passage section that guides fluid caused to flow from the pipe conduit opening section toward an observation window of the observing section are provided, the flow passage section includes a guide groove formed in a recess shape on the distal end face and a projecting section caused to project forward at a peripheral edge site of the pipe conduit opening section on the distal end face, and the projecting section is disposed at a peripheral edge site of the guide groove.

With the configuration, the distal end face of the insertion section to be inserted into a body to be examined is brought into contact with a body to be examined at a contacting observation time of the body to be examined. In this state, fluid such as stain solution caused to flow from the pipe conduit opening section for fluid supply is guided toward the observation window by the flow passage section. Further, by pushing the body to be examined by the projecting section disposed at the peripheral edge section of the guide groove, a gap is formed between the peripheral edge site of the pipe conduit opening section and the body to be examined, so that fluid such as stain solution is guided toward the observation window via the guide groove on the distal end face and the gap.

It is preferable that the flow passage section is a chamfered section provided on a ridge line of the pipe conduit opening section and a chamfer amount of the chamfered section is large only in a direction of the observation window.

With the configuration, in a state that the distal end face of the insertion section to be inserted into a body to be examined has been brought into contact with a body to be examined at a contacting observation time of the body to be examined, fluid such as stain solution caused to flow from the pipe conduit opening section for fluid supply is guided toward the observation window by the chamfered section which is provided on the ridge line of the pipe conduit opening section and whose chamfer amount is large only in the direction of the observation window.

It is preferable that the projecting section includes a pair of projecting pieces disposed on both sides of the guide groove.

With the configuration, in a state that the distal end face of the insertion section has been brought into contact with a body to be examined at a contacting observation time of the body to be examined, a gap is formed between the pair of projecting pieces on both the sides of the guide groove and the body to be examined by pushing the body to be examined by the pair of projecting pieces disposed on both the sides of the guide groove so that fluid such as stain solution is guided toward the observation window via the guide groove on the distal end face and the gap.

It is preferable that the projecting section is a nozzle for jetting cleaning water for cleaning the observation window.

With the configuration, in a state that the distal end face of the insertion section has been brought into contact with a body to be examined at a contacting observation time of the body to be examined, a gap is formed between a peripheral edge site of the pipe conduit opening section and the body to be examined by pushing the body to be examined by the jetting nozzle for cleaning water so that fluid caused to flow from the pipe conduit opening section is guided toward the observation window via the gap.

It is preferable that the pipe conduit opening section is caused to communicate with stain solution supplying means for supplying stain solution staining the body to be examined.

With the configuration, stain solution staining a body to be examined is supplied from the stain solution supplying means caused to communicate with the pipe conduit opening section and stain solution caused to flow from the pipe conduit opening section is guided toward the observation window by the flow passage section.

It is preferable that the pipe conduit opening section is connected to the stain solution supplying means for supplying stain solution staining the body to be examined and cleaning water supplying means for supplying cleaning water, respectively, and an operation section of the endoscope comprises control means for conducting selective switching between the stain solution supplying means and the cleaning water supplying means to drive the switched means.

With the configuration, by conducting selective switching between the stain solution supplying means and the cleaning water supplying means to drive the switched means by the control means of the operation section of the endoscope, selective switching is performed between a state that cleaning water is supplied from the cleaning water supplying means and a state that stain solution is supplied from the stain solution supplying means.

According to the present invention, an endoscope which can correctly stain a site where cell observation should be conducted when contacting observation of body tissue is conducted or an object is observed in a proximity state thereto and can improve workability of contacting observation can be provided.

The invention can be more fully understood from the following detailed description when taken in conjunction with the accompanying drawings, in which:
FIG. 1 is a schematic configuration diagram of a whole system of an endoscope according to a first embodiment of the present invention;
FIG. 2 is a front view of a distal end section of the endoscope according to the first embodiment;
FIG. 3 is a sectional view of the distal end section taken along line III - III in FIG. 2;
FIG. 4 is a vertical sectional view of a main section showing a configuration of an observation optical system of the endoscope according to the first embodiment;
FIG. 5 is a perspective view showing a flow passage section that guides fluid caused to flow from an opening section of a pipe conduit for fluid supply toward an observation window of an observing section in the endoscope according to the first embodiment;
FIG. 6 is a flowchart for explaining a working procedure conducted during observation conducted by the endoscope according to the first embodiment;
FIG. 7A is a vertical sectional view of a main section showing an observing state in a non-contacting state with body tissue using the endoscope according to the first embodiment;
FIG. 7B is a vertical sectional view of a main section showing an observing state in a contacting state with body tissue;
FIG. 8 is a front view of a distal end section showing an endoscope according to a second embodiment of the present invention;
FIG. 9 is a sectional view of the distal end section taken along line IX - IX in FIG. 8;
FIG. 10 is a vertical sectional view of a main section showing an observing state in a contacting state with body tissue using the endoscope according to the second embodiment;
FIG. 11 is a front view of a distal end section showing an endoscope according to a third embodiment of the present invention;
FIG. 12 is a sectional view of the distal end section taken along line XII - XII in FIG. 11; and
FIG. 13 is a front view of a distal end section showing an endoscope according to a fourth embodiment of the present invention.

### (First Embodiment)

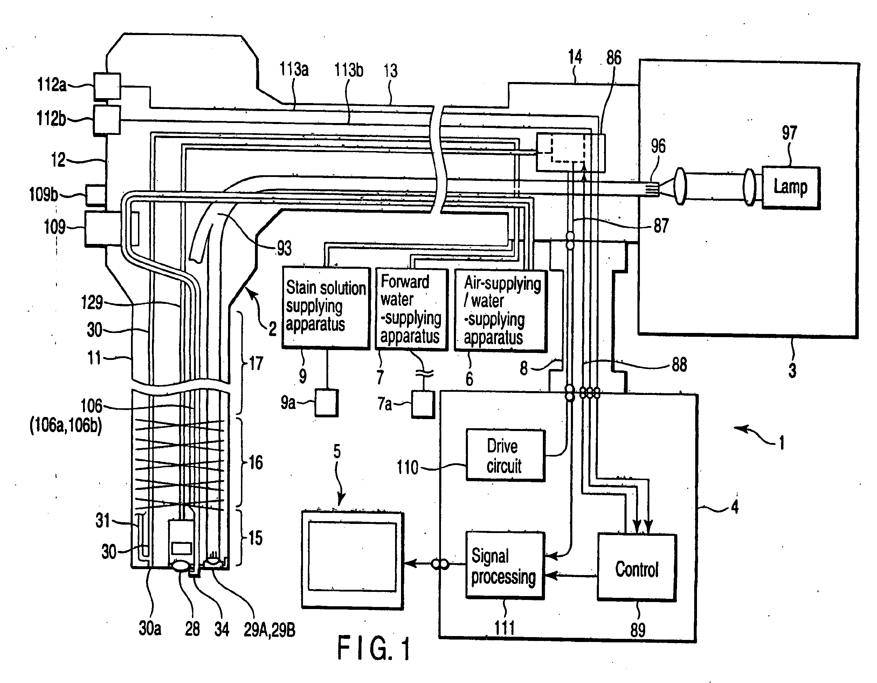

A first embodiment of the present invention will be explained below with reference to FIGS. 1 to 7B. FIG. 1 shows a schematic configuration of a whole endoscope system 1 according to the first embodiment. As shown in FIG. 1, an endoscope system 1 according to the embodiment is provided with an endoscope 2, a light source apparatus 3, a processor 4, a monitor 5, an air-supplying/water-supplying apparatus 6, a forward water-supplying apparatus 7, and a stain solution supplying apparatus 9. The light source apparatus 3 is illuminating means for supplying illumination light to the endoscope 2. The processor 4 is a signal processing apparatus for performing signal processing to the endoscope 2. The monitor 5 is connected to the processor 4. The air-supplying/water-supplying apparatus 6 performs air supply and water supply. The forward water-supplying apparatus 7 performs water-supplying forward. The stain solution supplying apparatus 9 supplies stain solution such as, for example, methylene blue.

The endoscope 2 includes a slender insertion section 11, an operation section 12, and a universal cable 13. The insertion section 11 is inserted into a body cavity. The operation section 12 is coupled to a proximal end of the insertion section 11. The universal cable 13 extends from a side section of the operation section 12. An end section of the universal cable 13 is provided with a connector 14. The connector 14 is attachably and detachably connected to the light source apparatus 3. Further, the connector 14 is connected to the processor 4 via a scope cable 8.

The insertion section 11 of the endoscope 2 includes a hard distal end section 15 formed at a distal end thereof, a bending section 16 formed at a proximal end of the distal end section 15, and a flexible tube section 17 with a flexibility formed so as to extend from a proximal end of the bending section 16 to the operation section 12.

A plurality of annular-shaped bending pieces (not shown) is provided rotatably on the bending section 16 of the endoscope 2 along an axial direction of the insertion section 11. Each bending piece is fixed with four pipe-shaped wire receivers on an inner peripheral face by means such as welding. Four wire receivers are fixed on the inner peripheral face of one bending piece at positions shifted from one another around an axis of the insertion section 16 about 90° apart.

The plurality of bending pieces is covered with a curved blade formed by knitting fine wires in a cylindrical shape so as to cover outer peripheries of the bending pieces. An outer skin 21 is covered with the bending blade so as to maintain water-tightness.

The outer skin 21 is arranged so as to cover the insertion section 11 comprising the distal end section 15, the bending section 16 and the flexible tube section 17 over its entire length integrally therewith. A distal end outer peripheral section of the outer skin 21 is firmly fixed by a spool bonding section 22 bonded after wound on the distal end section 15.

Four bending operation wires (not shown) for operating the bending section 16 in a bending manner are inserted in the insertion section 11. Distal end sections of the four bending operation wires are held and fixed to four fixing sections each comprising a fixing ring provided inside the distal end sections 15 while shifted from one another around an axis of the insertion section about 90° apart. Further, each of the four bending operation wires is inserted into each wire receiver on the inner peripheral face of the bending piece. Each of the four bending operation wires extends toward the operation section 12 on the proximal end side from the bending section 16 through the inside of the flexible tube section 17. The fixing ring is inserted and fitted to an inner peripheral side of a reinforcing ring 15b of the distal end section 15 described later.

Proximal end sections of the bending operation wires are coupled to a bending operation mechanism (not shown) provided inside the operation section 12 (see FIG. 1). A bending operation knob for four directions bending operations (not shown) that drives the bending operation mechanism is disposed on the operation section 12.

Four bending operation wires are alternately pulled and loosened according to operation of the bending operation knob so that the bending section 16 is operated in four directions in a bending manner. The four directions indicate four upward, downward, leftward, and rightward directions of an endoscope image displayed on the monitor 5 described later.

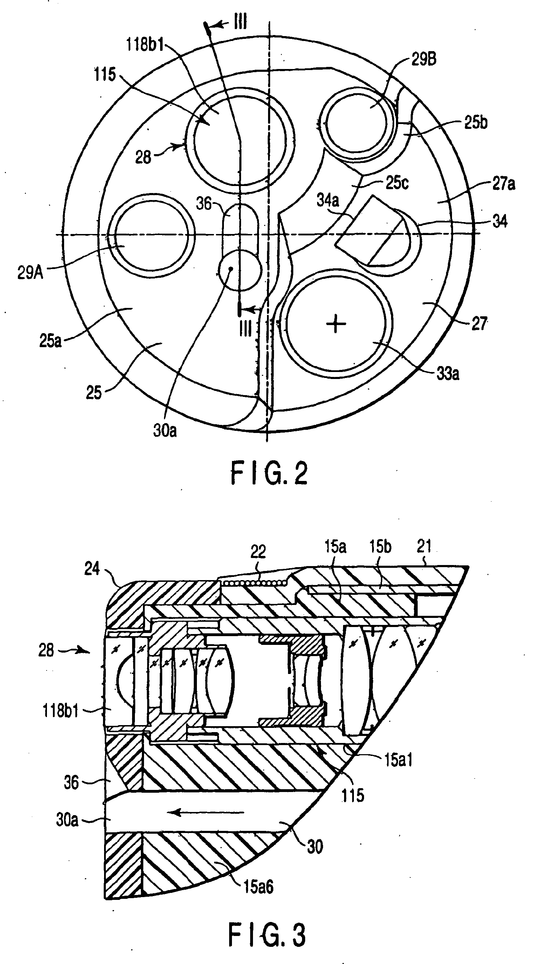

FIG. 2 shows an end face of the endoscope 2 of a direct-view type. In the endoscope 2 of a direct-view type, an end face of the distal end section 15 of the insertion section 11 is disposed in a direction orthogonal to an axial direction of the insertion section 11. One observing section 28, two (first and second) illuminating sections 29A and 29B, one distal end opening section 33a of an endo-therapy device insertion channel (also called a "forceps channel") 33, and one a air-supplying/water-supplying nozzle 34 and one pipe conduit opening section 30a of a pipe conduit (forward water-supplying channel) 30 for fluid supply are disposed at the end face of the distal end section 15. Thereby, the observing section 28 observes a body to be examined in a forward front direction. The first and second illuminating sections 29A and 29B emit illumination light. The pipe duct (forward water-supplying channel) 30 for fluid supplying supplies cleaning water for forward water supplying, stain solution, and the like.

FIG. 3 shows an internal configuration of a main section of the distal end section of the insertion section 11 of the endoscope 2 of the present embodiment. As shown in FIG. 3, a cylindrical member (a distal end hard member) 15a and the annular-shaped reinforcing ring 15b are disposed inside the distal end section 15 of the insertion section 11. The cylindrical member (distal end hard member) 15a is made from hard metal. The reinforcing ring 15b is fitted on an outer peripheral section of the cylindrical member 15a on a proximal end side thereof. A plurality of (six in the present embodiment) (first to sixth) hole sections 15a1 to 15a6 (only the first hole section 15a1 and the sixth hole section 15a6 are shown in FIG. 3) extending parallel to the axial direction of the insertion section 11 are formed in the cylindrical member 15a. A proximal end section of the reinforcing ring 15b is coupled to the bending pieces at the most-advanced bending piece.

Three (the first to third) hole sections 15a1 to 15a3 of the cylindrical member 15a are disposed at sites corresponding to the observing section 28 and the first and second illuminating sections 29A and 29B, respectively. Respective constituent elements of the observing section 28, the first illuminating section 29A, and the second illuminating section 29B are mounted on the first hole section 15al of the cylindrical member 15a, the second hole section 15a2 (not shown), and the third hole section 15a3 (not shown), respectively.

The remaining three (fourth to sixth) hole sections 15a4, 15a5, and 15a6 of the cylindrical member 15a are disposed at sites corresponding to a distal end opening section 33a of the endo-therapy device insertion channel 33, the air-supplying/water-supplying nozzle 34, and the opening section 30a of the pipe conduit 30 for fluid supply, respectively. Constituents elements of the pipe conduit of the endo-therapy device insertion channel 33 are coupled to the fourth hole section 15a4 (not shown) of the cylindrical member 15a. Similarly, constituent elements of the air-supplying/water-supplying nozzle 34 and the constituent elements of the pipe conduit 30 for forward water-supplying are coupled to the fifth hole sections 15a5 (not shown) of the cylindrical member 15a and the sixth hole section 15a6 of the cylindrical member 15a respectively.

A distal end cover 24 is attached to a distal end face of the cylindrical member 15a and a distal end side outer peripheral section of the cylindrical member 15a in a state of being fitted thereon. As shown in FIG. 2, two-stage step sections 25 and 27 including a projecting step section (projecting section) 25 projecting forward and a lower stage section 27 lower than the projecting step section 25 by one step are formed on the distal end cover 24. An end face of the projecting step section 25 is formed by a flat face 25a orthogonal to the axial direction of the insertion section 11. A projecting face is formed by the flat face 25a of the projecting step section 25.

In the present embodiment, the flat face 25a of the projecting step section 25 is formed to have an area of about 2/3 of a circular shape of the whole front face of the distal end cover 24. The observing section 28, two (first and second) illuminating sections 29A and 29B, and one pipe conduit opening section 30a of the pipe conduit 30 for fluid supply such as cleaning water for forward water-supplying or stain solution are disposed on the flat face 25a of the projecting step section 25. Two illuminating sections 29A and 29B are disposed on both sides of the observing section 28. The pipe conduit opening section 30a for fluid supply is disposed near the observing section 28.

The lower step section 27 includes a flat face 27a approximately parallel to the flat face 25a of the projecting step section 25. A distal end opening section 33a of the endo-therapy device insertion channel 33 disposed inside the insertion section 11 and the air-supplying/water-supplying nozzle 34 are disposed on the flat face 27a of the lower step section 27.

Furthermore, an inclined face 25b with an inclined angle, for example, about 45° and a fluid guide face 25c having an inclined angle smaller than that of the inclined face 25b are formed on a wall section between the lower step section 27 and the projecting step section 25. The fluid guide face 25c is disposed between the air-supplying/water-supplying nozzle 34 of the lower step section 27 and the observing section 28 of the projecting step section 25. The fluid guide face 25c is formed by a gentle inclined face having an inclined angle, for example, about 18°.

The air-supplying/water-supplying nozzle 34 is a pipe-like member bent in an approximately L shape. The air-supplying/water-supplying nozzle 34 is disposed such that a distal end section thereof is directed to the observing section 28. A jetting port 34a at the distal end opening section of the air-supplying/water-supplying nozzle 34 is disposed to face the fluid guide face 25c. The air-supplying/water-supplying nozzle 34 is connected to an air-supplying/water-supplying pipe conduit 106 joined together with a distal end side of the air-supplying/water-supplying nozzle 34, and a proximal end side of the air-supplying/water-supplying pipe conduit 106 is branched into an air-supplying pipe conduit 106a and a water-supplying pipe conduit 106b.

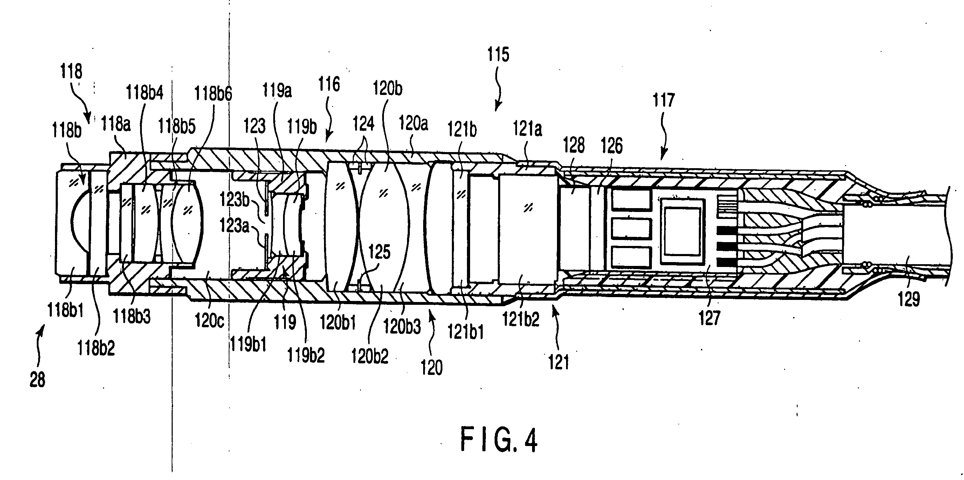

An observation optical system 115 of a separation and contact two-way type is provided on the observing section 28. FIG. 4 shows an internal structure of the observation optical system 115 of a separation and contact two-way type. The observation optical system 115 of a separation and contact two-way type includes a zoom lens section 116 provided with a zoom optical system that can continuously change an observation magnification ratio from Tele (enlargement) position to Wide (wide-angle) position and an electric part section 117.

The zoom lens section 116 further includes 4 (first to fourth) section configuration bodies 118 to 121. The first section configuration body 118 includes a first lens frame 118a and a first lens group 118b configuring an objective lens. As shown in FIG. 4, the first lens group 118b includes six (first to sixth) lenses 118b1 to 118b6. Here, the first lens 118b1 which is an observation lens forming an observation window of the observing section 28 is disposed at the most-advanced section of the first lens frame 118a. A distal end section (distal end observation face) of the first lens 118b1 is bonded and fixed to the first lens frame 118a, for example, by black color adhesive in a state that it has been projected forward beyond the distal end section of the first lens frame 118a. Thereby, a section of a distal end outer peripheral face of the first lens 118b1 that has been projected forward beyond the distal end section to the first lens frame 118a is held in a state that it has been covered with the black color adhesive.

The second section configuration body 119 is a movable optical section for zooming which can advance and retreat in a photographing optical axis direction. The second section configuration body 119 includes a second lens frame (sliding lens frame) 119a and a second lens group (zoom lens) 119b. The second lens group 119b includes two (first and second) lenses 119b1 and 119b2.

The third section configuration body 120 includes a third lens frame 120a and a third lens group 120b. A guide space 120c where the second section configuration body 119 is held to be capable of advancing and retreating in the photographing optical axis direction is provided inside the third lens frame 120a on a distal end side of the third lens frame 120a. A third lens group 120b is disposed behind the guide space 120c. The third lens group 120b includes three (first to third) lenses 120b1 to 120b3.

The fourth section configuration body 121 includes a fourth lens frame 121a and a fourth lens group 121b. The fourth lens group 121b includes two (first and second) lenses 121b1 and 121b2.

A distal end section of an operation wire (not shown) for operating the second section configuration body 119 to advance and retreat in the photographing optical axis direction is fixed to the second lens frame 119a of the second section configuration body 119. When an operation lever for zooming (not shown) provided on the operation section of the endoscope is operated by a user, the operation wire is driven to advance and retreat in the photographing optical axis direction. At this time, the second section configuration body 119 that is the zoom optical system is moved forward (Wide [wide-angle] position direction) according to operation by which the operation wire is pushed out in a distal end direction thereof. Further, the second section configuration body 119 which is the zoom optical system is moved toward a near side (Tele [enlargement] position direction) according to operation by which the operation wire is pulled toward the near side. Here, a state that the second section configuration body 119 has been moved to a position except for the rearmost end position inside the guide space 120c of the third section configuration body 120 is set to a range (an ordinary observation mode) where observation is conducted in a non-contacting state with a body to be examined. A state that the second section configuration body 119 has been moved to the rearmost end position inside the guide space 120c is set to an observation position (an object contact observation mode where a monitor observation magnification ratio is in a range of about 200 to 1000 times) where observation is conducted in a contacting state with the body to be examined. Thereby, switching can be performed between the observation position (the ordinary observation mode) where observation is performed in the non-contacting state of the second section configuration body 119 with a body to be examined and the observation position (the object contact observation mode) where observation is performed in the contacting state of the second section configuration body 119 with a body to be examined according to an operating lever for zooming (not shown).

A brightness diaphragm 123 is provided in the second lens frame 119a in the sliding second section configuration body 119 for zooming. The brightness diaphragm 123 is disposed on a front face side of the first lens 119b1 held by the second lens frame 119a. In the brightness diaphragm 123, an opening section 123b that allows transmission of light is provided at a central section of a light blocking sheet 123a.

A plurality of (two in the present embodiment) spacer rings 124 serving as positioning members for determining a lens distance between the first lens 120b1 and the second lens 120b2 is interposed in the third section configuration body 120. A flare diaphragm 125 for preventing optical flare is interposed between the two spacer rings 124.

An electric part section 117 is continuously provided at a rear end section of the fourth section configuration body 121. The electric part section 117 includes an imaging device 126 such as a charge coupled device (CCD) or a complementary metal-oxide semiconductor (CMOS) device and a circuit board 127. Further, a cover glass 128 is provided on a light receiving face side of a front face of the imaging device 126.

The cover glass 128 of the electric part section 117 is fixed parallel to the second lens 121b2 of the fourth section configuration body 121. Thereby, the observation optical system 115 of a separation and contact two-way type where the zoom lens section 116 and the electric part section 117 are integrated with each other is formed.

The circuit board 127 includes electric parts and a wiring pattern, and is connected with distal end sections of a plurality of signal wires in a signal cable 129 by means such as soldering. Further, respective outer peripheral sections of the cover glass 128, the imaging device 126, the circuit board 127 and a distal end section of the signal cable 129 are integrally covered with insulating sealing resin or the like.

An optical image focused on the imaging device 126 from the zoom lens section 116 is photoelectrically converted to electric image signals by the imaging device 126, and the electric image signals are output to the circuit board 127. Electric signals of the optical image output from the circuit board 127 are transmitted to the processor 4 which is an electric device following the circuit board 127 via the signal cable 129.

As shown in FIG. 1, the signal cable 129 of the imaging device 126 extends into the connector 14 sequentially via the insides of the insertion section 11, the operation section 12 and the universal cable 13. A relay board 86 is incorporated in the connector 14. A proximal end section of the signal cable 129 is connected to the relay board 86. The signal cable 129 is connected to a signal cable 87 via the relay board 86 in the connector 14.

Further, the relay board 86 of the connector 14 is connected to a control circuit 89 in the processor 4 via the signal cable 87 in the connector 14 and a signal wire 88 in the scope cable 8.

A drive circuit 110, a signal processing circuit 111, and the control circuit 89 are provided in the processor 4. The drive circuit 110 drives the imaging device 126 of the observation optical system 115. The signal processing circuit 111 performs signal processing to an imaging signal output from the imaging device 126 via the relay board 86. The control circuit 89 controls an operating state of the signal processing circuit 111 and the like.

As shown in FIG. 3, the observation optical system 115 of a separation and contact two-way type is mounted and fixed to the cylindrical member 15a in a state of being inserted into the first hole section 15a1 by a screw (not shown). Here, a front end section of the first lens 118b1 of the first section configuration body 118 is fixed in a state that it has been caused to project forward beyond the position of the flat face 25a of the projection step section 25 by a proper length L1, for example, about 0.05 mm.

The first and second illuminating sections 29A and 29B have substantially the same configuration. A distal end section of a light guide 93 transmitting illumination light to a rear end section of an illumination lens is disposed at each of the first and second illuminating sections 29A and 29B. The light guide 93 has a distal end section covered with a cylindrical member and is covered with an outer skin formed by bundling a plurality of glass fiber and a protective tube which is a core material.

The light guide 93 extends into the connector 14 sequentially via inside sections of the insertion section 11, the operation section 12 of the endoscope 2 and the universal cable 13. A proximal end section side of the light guide 93 is connected to an incident end section 96 of a light guide connector projecting from the connector 14. The incident end section 96 of the light guide connector is attachably and detachably connected to the light source apparatus 3.

In the present embodiment, the light guide 93 is branched into two light guide pieces inside the operation section 12 of the endoscope 2 and the two branched light guide pieces are inserted into the insertion section 11. Distal end sections of the respective two-branched light guide pieces of the light guide 93 are disposed opposite to each other near two illumination windows provided in the distal end cover 24, namely, back faces of respective illumination lenses of the first illuminating section 29A and the second illuminating section 29B, and the two-branched light guide pieces are fixed to rear end sections of the second hole section 15a2 and the third hole section 15a3 of the cylindrical member 15a, for example, by screw clamping.

Illumination light from an illumination lamp 97 of the light source apparatus 3 is emitted on an incident end section 96 of the light guide connector and illumination light guided through the light guide 93 is emitted in front of the endoscope 2 via respective illumination lenses of the first illuminating section 29A and the second illuminating section 29B.

A distal end section of a communicating tube communicating with the endo-therapy device insertion channel 33 is inserted and fitted into the fourth hole section 15a4 formed in the cylindrical member 15a of the distal end section 15 from a proximal end section side of the fourth hole section 15a4. A distal end section of the communicating tube is caused to project rearward beyond the cylindrical member 15a. The distal end section of the endo-therapy device insertion channel 33 is coupled to the proximal end section of the communicating tube. A distal end of the endo-therapy device insertion channel 33 is caused to communicate with a distal end opening section 33a of the distal end cover 24 via a communicating tube.

The endo-therapy device insertion channel 33 is branched in the near the proximal end of the insertion section 11 and one of the branched channels is inserted up to an endo-therapy device insertion port (not shown) arranged in the operation section of the endoscope. The other of the branched channels communicates with a suction channel through insides of the insertion section 11 and the universal cable 13 and a proximal end thereof is connected to suction means (not shown) via the connector 14.

A distal end section of an approximately cylindrical tube member is inserted from a rear end section into the sixth hole section 15a6 formed in the cylindrical member 15a of the distal end section 15 and fitted into the sixth hole section 15a6. A proximal end section of the tube member projects rearward the cylindrical member 15a, and a distal end section of the forward water-supplying pipe conduit 30 is coupled to the proximal end section of the tube member. Incidentally, the distal end section of the forward water-supplying pipe conduit 30 is connected and fixed to the proximal end section of the tube member by the spool covering the proximal end section of the tube member.

The forward water-supplying pipe conduit 30 is inserted up to the connector 14 though the insertion section 11, and the operation section 12 and the universal cable 13 of the endoscope 2, and it is connected to the forward water-supplying apparatus 7. A forward water-supplying button 109b is interposed in an intermediate section of the forward water-supplying pipe conduit 30 at the operation section 12 of the endoscope 2.

When the forward water-supplying button 109b is operated by an operator, liquid such as sterile water is sprayed from the opening section 30a of the distal end cover 24 of the insertion section 11 in an insertion direction to a body cavity. Thereby, body fluid adhered on a body to be examined in a body cavity can be cleaned. Incidentally, as shown in FIG. 1, a foot switch 7a is connected to a cable extending from the forward water-supplying apparatus 7, and a user can also spray liquid such as sterile water from the opening section 30a of the distal end cover 24 of the insertion section 11 in an insertion direction to a body cavity according to operation of the foot switch 7a.

Further, a stain solution feeding pipe conduit 31 for supplying stain solution is disposed in the insertion section 11 parallel to the forward water-supplying pipe conduit 30. A distal end section of the stain solution feeding pipe conduit 31 joins the forward water-supplying pipe conduit 30. The stain solution supplying pipe conduit 31 is inserted up to the connector 14 through the insertion section 11, and the operation section 12 and the universal cable 13 of the endoscope 2, and is connected to the stain solution supplying apparatus 9. Incidentally, forward water-supplying button 109b at the operation section 12 is configured as a button of two-stage operation type, for example. Liquid such as sterile water is sprayed from the opening section 30a of the distal end cover 24 according to button operation of the first stage, while switching to a state that stain solution is supplied from the stain solution supplying apparatus 9 to the opening section 30a of the distal end cover 24 is conducted according to button operation of the second stage. A foot switch 9a is connected to a cable extending from the stain solution supplying apparatus 9. A user can also supply stain solution from the stain solution supplying apparatus 9 to the opening section 30a of the distal end cover 24 of the insertion section 11 via the stain solution supplying pipe conduit 31 according to operation of the foot switch 9a.

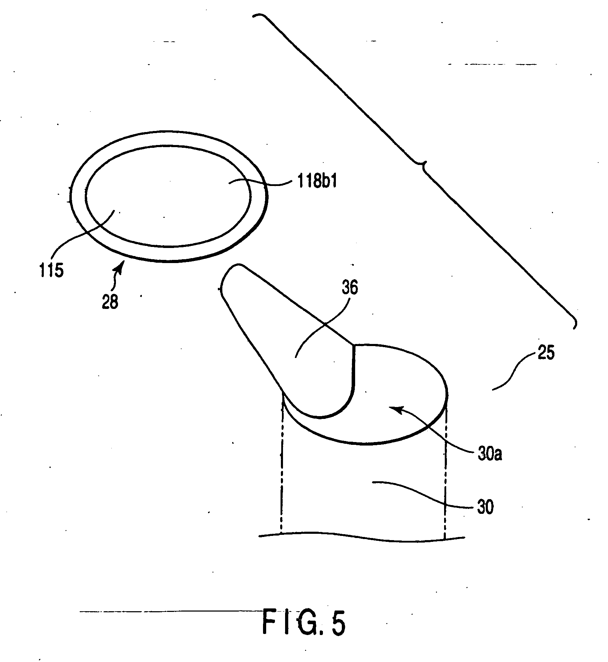

As shown in FIG. 5, a guide groove (flow passage section) 36 that guides fluid caused to flow from the opening section 30a of the forward water-supplying pipe conduit 30 in a direction of the observation window of the observing section 28 is provided on the flat face 25a of the projecting step section 25 of the distal end cover 24. The guide groove 36 is formed in a recess shape on the flat face 25a of the projecting step section 25. Incidentally, the flow passage section that guides fluid caused to flow from the opening section 30a of the forward water-supplying pipe conduit 30 in a direction of the observation window of the observing section 28 may be configured by not only the guide groove 36 but also the chamfered section provided on the ridge line of the opening section 30a of the forward water-supplying pipe conduit 30, and such a configuration can be adopted that a chamfer amount of the chamfered section is large only in the direction of the observation window of the observing section 28.

Incidentally, control switches 112a and 112b, an air-supplying/water-supplying button 109, a bending operation knob (not shown), a zoom lever (not shown) for conducting zoom operation of the observation optical system 115, the forward water-supplying button (not shown), and the endo-therapy device insertion port (not shown) described above are provided on the operation section 12 of the endoscope 2.

An endoscope image from the imaging device 126 of the observation optical system 115 is displayed on the monitor 5 according to input of a video signal output from the signal processing circuit 111 in the processor 4. Upward and downward directions of the monitor 5 coincides with a vertical transfer direction of the CCD device or the CMOS device in the imaging device 126, while leftward and rightward directions thereof coincide with a horizontal transfer direction of the CCD device or the CMOS device in the imaging device 126. That is, upward, downward, leftward, and rightward directions of an endoscope image photographed by the imaging device 126 in the observation optical system 115 coincide with upward, downward, leftward, and rightward directions of the monitor 5.

Upward, downward, leftward, and rightward directions of the bending section 16 of the insertion section 11 are determined so as to correspond to upward, downward, leftward, and rightward directions of an endoscope image displayed on the monitor 5. That is, four bending operation wires 23 inserted inside the bending section 16 are pulled or loosed according to predetermined operations of the bending operation knob provided on the operation section 12, as described above, so that the bending section 16 can be freely bend in four directions of upward, downward, leftward, and rightward directions corresponding to upward, downward, leftward, and rightward directions of an image displayed on the monitor 5.

Next, operation of the endoscope with the configuration described above will be explained. At a time of use of the endoscope according to the present embodiment, after setting of the endoscope system is completed, work for inserting the endoscope into a body of a patient is started. At an insertion work time of the endoscope, a user (operator) inserts the insertion section 11 of the endoscope 2 with the observation optical system 115 of a separation and contact two-way type of the observing section 28 into a body cavity and sets an observation mode to an ordinary observation mode that allows observation of an affected site to be diagnosed or the like in advance. In the ordinary observation mode, the second section configuration body 119 that is the zoom optical system of the observation optical system 115 is set to a range of an ordinary observation position where observation is conducted in a non-contacting state with a body to be examined.

Illumination lights of, for example, RGB are frame-sequentially supplied to the light guide 93 from the illumination lamp 97 of the light source apparatus 3. Thereby, the affected side or the like in the body cavity of the patient is illuminated via the first illuminating section 29A and the second illuminating section 29B. In synchronism with the illumination, the drive circuit 110 outputs a CCD drive signal to the imaging device 126.

A subject of the illuminated affected site or the like is focused on a light receiving face of the imaging device 126 through the zoom lens section 116 of the observation optical system 115 at the ordinary observation position to be photoelectrically converted. The imaging device 126 outputs the photoelectrically-converted signal according to application of a drive signal. The signal is input into the signal processing circuit 111 in the processor 4 via the signal cable 129. After the signal input into the signal processing circuit 111 is digitized internally, temporarily stored in memory for R, G, and B temporarily stored.

Thereafter, signals stored in the memory for R, G, and B are simultaneously read to form synchronized R, G, and B signals, and the R, G, and B signals thus synchronized are converted to analog R, G, and B signals so that the R, G, and B signals are color-displayed on the monitor 5. Thereby, ordinary observation where an object to be observed spaced from the first lens 118b1 of the zoom lens section 116 of the observation optical system 115 is observed over a wide range is conducted using the zoom lens section 116 of the observation optical system 115 at an ordinary observation position.

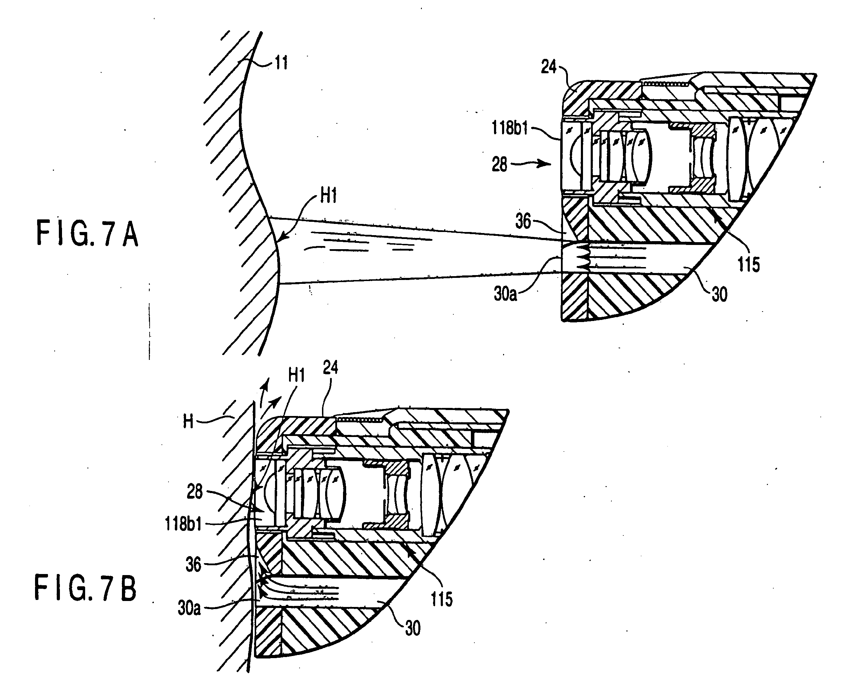

When body fluid adheres to a site to be examined in a body cavity during the ordinary observation so that the site becomes contaminated, button operation of the first stage to the forward water-supplying bottom is performed. As shown in FIG. 7A, liquid such as sterile water is sprayed in the insertion direction to a body cavity from the opening section 30a of the distal end cover 24 of the insertion section 11 at an operation time of the forward water-supplying button. Thereby, body liquid which has adhered on the site to be examined in the body cavity or the like can be cleaned.

The observation conducted by the zoom lens section 116 of the observation optical system 115 at the ordinary observation position is continued until the distal end section 15 of the endoscope 2 inserted into the body of the patient is guided up to the targeted observation object site H1. In a state that the distal end section 15 of the endoscope 2 has approached the targeted observation subject site H1, switching to an object contacting observation mode with high magnification ratio is conducted according to operation of the operation lever for zooming by the user. At this time, the second section configuration body 119 of the zoom lens section 116 of the observation optical system 115 is switched to a state that it has been moved to the rearmost position of the guide space 120c, so that switching to an object contacting observation position with high magnification ratio where observation is performed in a contacting state with a body to be examined is performed.

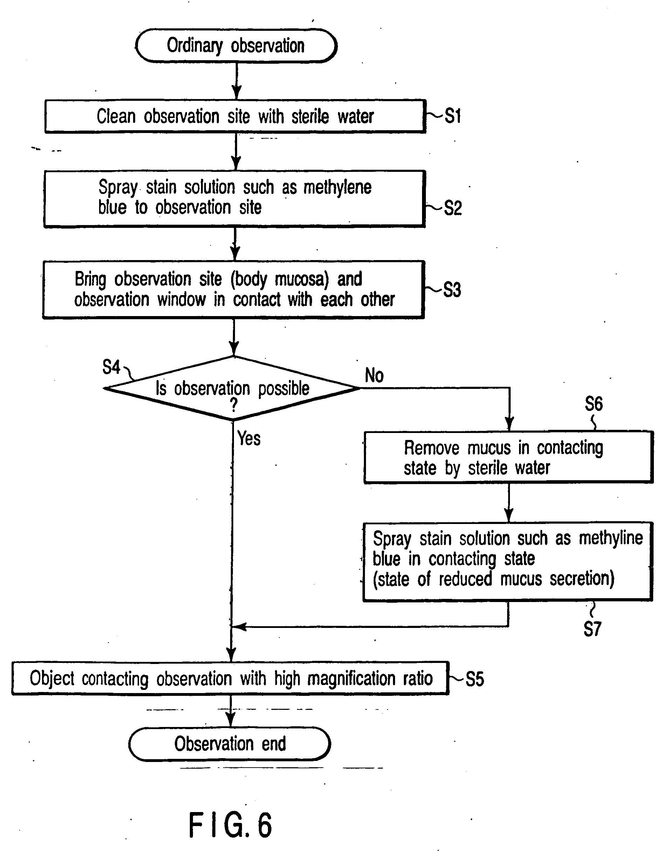

Observation is performed according to a procedure shown by a flowchart in FIG. 6 during contacting observation of a body to be examined in the contacting observation mode with high magnification ratio. First, button operation of the first stage to the forward water-supplying button is performed at a position spaced from the surface of body tissue H, as shown in FIG. 7A. Liquid such as sterile water is sprayed from the opening section 30a of the distal end cover 24 of the insertion section 11 to a targeted observation object site H1 in the insertion direction to a body cavity (step S1). Thereby, body liquid which has adhered to a site to be examined inside the body cavity or the like is cleaned.

Subsequently, button operation of the second step to the forward water-supplying button is performed. At this time, stain solution (dye) such as methylene blue is sprayed to the targeted observation object site H1 from the opening section 30a of the distal end cover 24 of the insertion section 11 in the insertion direction to the body cavity (step S2). Thereby, the site interested is stained in advance so that a contour of a cell can be observed sharply.

Thereafter, at the next step S3, the flat face 25a of the projecting step section 25 of the distal end section 15 of the insertion section 11 is pushed against a surface of the body tissue H which is an object, as shown in FIG. 7B. At this time, the flat face 25a of the projecting step section 25 of the distal end cover 24 is mainly pushed against the surface of the body tissue H while a non-projecting face such as the flat face 27a of the lower step section 27 except for the flat face 25a is held in a non-contacting state with the surface of the body tissue H. Therefore, the first lens 118b1 and the illumination lenses of the illumination sections 29A and 29B on the distal end of the observation optical system 115 disposed on the flat face 25a of the projecting step section 25 are brought into pressure-contact with the surface of the body tissue H such as cell tissue at the observation object site H1.

In this state, determination is made about whether or not enlargement observation of the surface of the body tissue H such as cell tissue to be observed can be performed at a high magnification ratio (step S4). When the determination that the enlargement observation is possible is made at the step S4, object contacting observation at a high magnification ratio where cell tissue to be observed or the like is observed at a high magnification ratio is performed (step S5). Thereafter, the observation is terminated.

When the determination that the enlargement observation is impossible is made at the step S4, button operation of the first step to the forward water-supplying button is performed in a state that the flat face 25a of the projecting step section 25 has been mainly pushed against the surface of the body tissue H. Liquid such as sterile water is sprayed from the opening section 30a of the distal end cover 24 in the insertion direction to the body cavity (step S6). Thereby, body fluid or the like that has adhered to the site to be examined in the body cavity is cleaned.

Thereafter, button operation of the second step to the forward water-supplying button is performed. Stain solution (dye) such as, for example, methylene blue is sprayed from the opening section 30a of the distal end cover 24 of the insertion section 11 in the insertion direction to the body cavity so that the targeted observation object site H1 is stained by the stain solution (step S7). At this time, fluid such as stain solution caused to flow from the opening section 30a of the distal end cover 24 is guided in the direction of the observation window of the observing section 28 by the guide groove 36. Therefore, stain solution such as methylene blue is surely sprayed in a gap between the first lens 118b1 of the observation window of the observing section 28 and the surface of the body tissue H. Thereby, the site interested is stained in advance so that a contour of a cell can be observed sharply. In this state, the procedure proceeds to step S5 where object contacting observation with high magnification ratio where cell tissue to be observed or the like is observed at a high magnification ratio is performed. Thereafter, the observation is terminated.

At a cell observation time of body tissue H conducted by the observation optical system 115 of an object contacting type, illumination light is irradiated onto body tissue H such as cell tissue through illumination lenses of the first and second illumination sections 29A and 29B. At this time, a section of illumination light irradiated onto the body tissue H such as cell tissue is transmitted into inside of the body tissue H such as cell tissue to diffuse to surrounding section of abutting faces of the illumination lenses of the first and second illumination sections 29A and 29B. Therefore, illumination light is emitted on a surrounding section of the body tissue H such as cell tissue in front of the first lens 118b1 of the observation optical system 115. Thereby, illumination light is emitted on a section to be observed by the first lens 118b1 of the observation optical system 115 that has been pushed against the surface of the body tissue H such as cell tissue, so that light from the body tissue H such as cell tissue is focused on the light receiving face of the imaging device 126 through the zoom lens section 116 of the observation optical system 115 to be photoelectrically converted.

The imaging device 126 outputs a photoelectrically-converted signal. In this case, the photoelectrically-converted signal is output from the imaging device 126 after signal-amplified inside the imaging device 126. The signal is input to the signal processing circuit 111 in the processor 4 via the signal cable 129.

After the signal input to the signal processing circuit 111 is digitized internally, respective R, G, and B elements of the signal are simultaneously stored in the memory for R, G, and B, for example. Thereafter, signals stored in the memory for R, G, and B are simultaneously read to form synchronized R, G, and B signals, and the R, G, and B signals thus synchronized are converted to form analog R, G, and B signals so that the R, G, and B signals are displayed on the monitor 5. Thereby, observation of the body tissue H such as cell tissue in front of the first lens 118b1 of the observation optical system 115 can be performed in the observation mode at a high magnification ratio using the observation optical system 115 of an object contacting type.

With the above configuration, the following effects can be obtained. That is, according to the present embodiment, the guide groove 36 for guiding fluid caused to flow from the opening section 30a of the forward water-supplying pipe conduit 30 in the direction of the observation window (the first lens 118b1 of the observation optical system 115) of the observing section 28 is provided on the flat face 25a of the projecting step section 25 of the distal end cover 24. Thereby, even when stain solution is sprayed from the opening section 30a of the distal end cover 24 of the insertion section 11 in a state that the flat face 25a of the projecting step section 25 has been mainly pushed against the surface of the body tissue H, stain solution caused to flow from the opening section 30a of the distal end cover 24 is guided in the direction of the observation window of the observing section 28 by the guide groove 36. Therefore, since stain solution such as methylene blue is surely sprayed in the gap between the first lens 118b1 of the observation window of the observing section 28 and the surface of the body tissue H in a state that the observation window (the first lens 118b1 of the observation optical system 115) of the observing section 28 has been pushed against the surface of the body tissue H at the object contacting observation time with a high magnification ratio where cell tissue to be observed or the like is observed at a high magnification ratio, a site interested is stained so that a contour of a cell can be observed sharply.

Though a subject (stomach or the like) secreting much mucus must be stained after the mucus has been removed, secretion of mucus can be reduced by bringing the distal end section 15 of the endoscope 2 in contact with a mucosa so that staining can be performed more stably.

Further, when the distal end section 15 of the endoscope 2 is not brought into contact with a mucosa, mucus removal conducted by utilizing water-supply or the like in the same manner as the ordinary forward water-supply and spraying of stain solution are possible.

### (Second Embodiment)

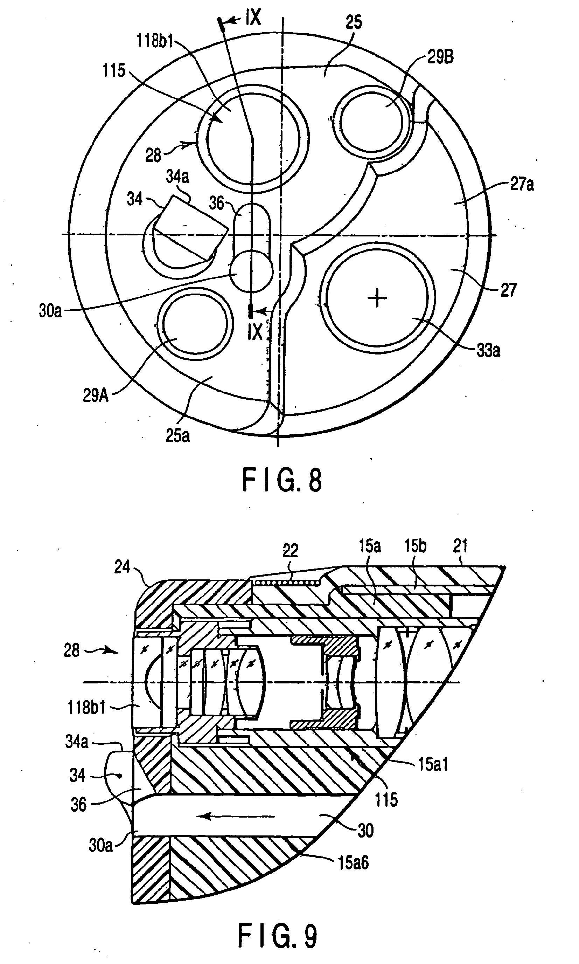

FIGS. 8 to 10 show a second embodiment of the present invention. In the second embodiment, a configuration of the distal end section 15 of the insertion section 11 of the endoscope 2 according to the first embodiment (see FIGS. 1 to 7B) is changed in the following manner.

That is, in an endoscope 2 according to the present embodiment, an air-supplying/water-supplying nozzle 34 is disposed at a flat face 25a of a projecting step section 25 of a distal end cover 24 at a distal end section 15 of an insertion section 11. As shown in FIG. 8, the air-supplying/water-supplying nozzle 34 is disposed adjacent to the near a guide groove 36. Further, the air-supplying/water-supplying nozzle 34 is attached so as to project forward from the flat face 25a of the projecting step section 25 of the distal end cover 24, as shown in FIG. 9.

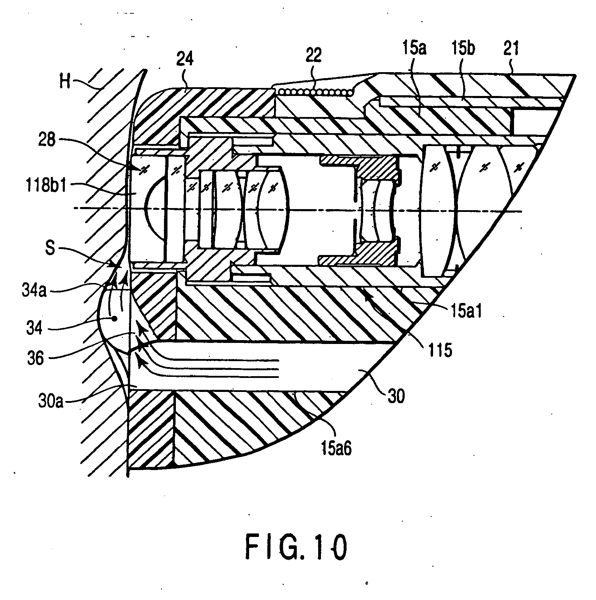

With the configuration, the following effect can be obtained. That is, in the endoscope 2 according to the second embodiment, at a contacting observation time with a high magnification ratio where cell tissue to be observed or the like is observed at a high magnification ratio in a state that an observation window (a first lens 118b1 of an observation optical system 115) of an observing section 28 has been pushed against a surface of body tissue H, a body to be examined can be pushed in by the air-supplying/water-supplying nozzle 34, as shown in FIG. 10. Thereby, a gap S can be formed between the first lens 118b1 of the observation window of the observing section 28 and the surface of the body tissue H. Therefore, at the contacting observation time with a high magnification ratio, stain solution such as methylene blue caused to flow from an opening section 30a of a distal end cover 24 can be guided in a direction of the observation window of the observing section 28 by the guide groove 36, and stain solution guided by the guide groove 36 is surely sprayed in the gap S between the first lens 118b1 of the observation window of the observing section 28 and the surface of the body tissue H. As a result, a site interested is stained so that a contour of a cell can be observed sharply even at the contacting observation time with a high magnification ratio.

In the embodiment, especially, since the air-supplying/water-supplying nozzle 34 is disposed adjacent to the near the guide groove 36, a flow passage section of the stain solution can be secured more stably and staining can be carried out more stably even in a contacting state with a living body.

In the present embodiment, since the air-supplying/water-supplying nozzle 34 generally disposed at the distal end section 15 of the insertion section 11 is utilized as a projecting section for pushing in a body to be examined, it is unnecessary to provide a projecting section which is utilized only for pushing in a body to be examined. Therefore, the number of constituent parts for the endoscope 2 can be reduced, which results in cost reduction.

### (Third Embodiment)

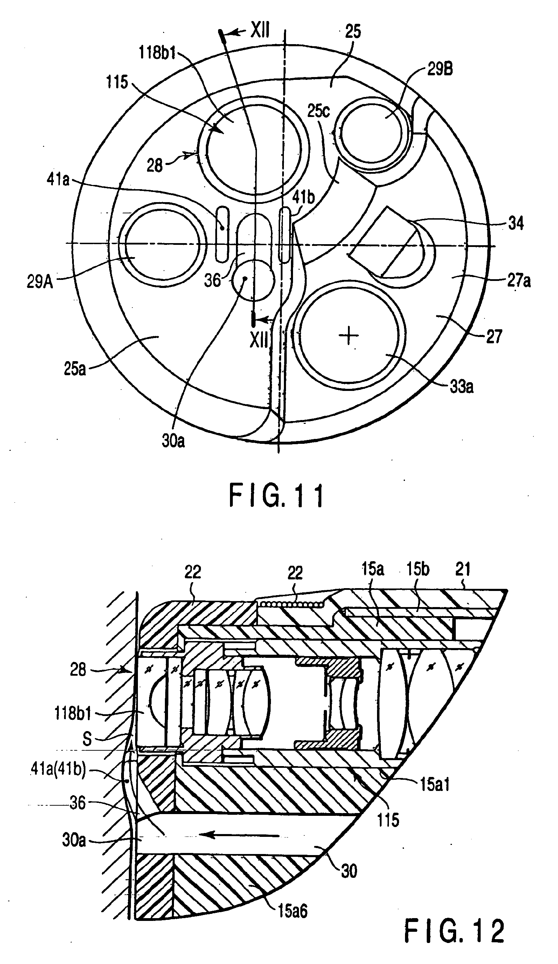

FIGS. 11 and 12 show a third embodiment of the present invention. In the third embodiment, a pair of projecting sections 41a and 41b is provided in a protruding manner on both sides of a guide groove 36 instead of the air-supplying/water-supplying nozzle 34 according to the second embodiment (see FIGS. 8 to 10), as shown in FIG. 11.

With the configuration described above, the following effect can be obtained. That is, in an endoscope 2 according to the third embodiment, at a contacting observation time with a high magnification ratio where cell tissue to be observed or the like is observed at a high magnification ratio in a state that an observation window (a first lens 118b1 of an observation optical system 115) of an observing section 28 has been pushed against a surface of body tissue H, a body to be examined can be pushed in by the pair of projecting sections 41a and 41b, as shown in FIG. 12. Thereby, a gap S can be formed between the first lens 118b1 of the observation window of the observing section 28 and the surface of the body tissue H. Therefore, at the contacting observation time with a high magnification ratio, stain solution caused to flow from an opening section 30a of a distal end cover 24 can be guided in a direction of the observation window of the observing section 28 by the guide groove 36, and stain solution such as methylene blue guided by the guide groove 36 is surely sprayed in the gap S between the first lens 118b1 of the observation window of the observing section 28 and the surface of the body tissue H. As a result, a site interested is stained so that a contour of a cell can be observed sharply even at the contacting observation time with a high magnification ratio.

### (Fourth Embodiment)

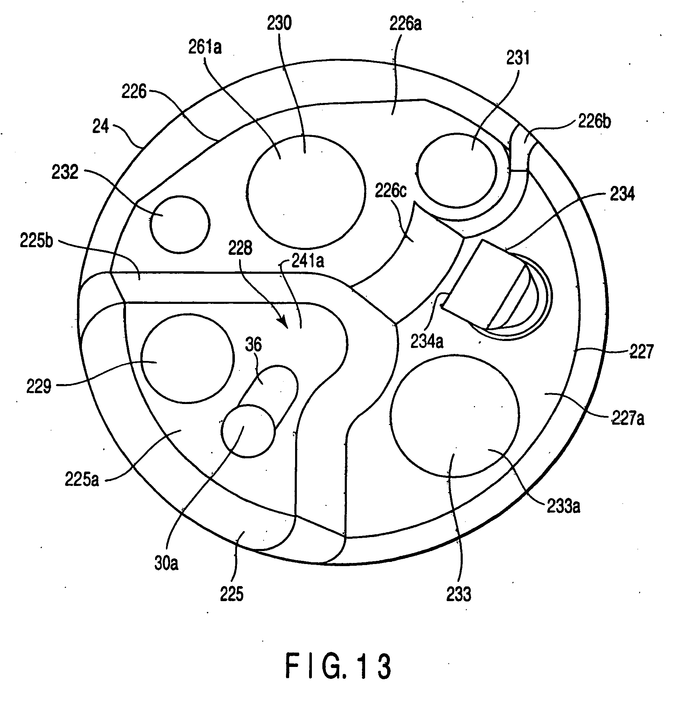

FIG. 13 shows a fourth embodiment of the present invention. In the present embodiment, the configuration of the distal end section 15 of the insertion section 11 of the endoscope 2 according to the first embodiment (see FIGS. 1 to 7B) is changed in the following manner.

That is, in an endoscope 2 according to the present embodiment, step sections of three steps 225, 226, and 227 including a projecting step section 225 projecting forward, a middle step section 226 lower than the projecting step section 225 by one step, and a lower step section 227 lower than the middle step section 226 by one step are formed on a distal end cover 24 disposed on the distal end section 15 of the insertion section 11, as shown in FIG. 13. Here, an end face of the projecting step section 225 is formed by a flat face 225a orthogonal to an axial direction of the insertion section 11. A projecting face is formed by the flat face 225a of the projecting step section 225.

In the present embodiment, the flat face 225a of the projecting step section 225 is formed to have an area of about 1/4 of a whole circular front face of the distal end cover 24. That is, the flat face 225a is formed at a lower half section of the whole circular front face of the distal end cover 24 on a left side section regarding a centerline connecting an upper side and a lower side in FIG. 13.

A first lens 241a that is an observation lens of a first imaging section (a first observing section) 228 of an object contacting type and a first illumination window (a first illumination section) 229 are disposed on the flat face 225a of the projecting step section 225. The first imaging section 228 is disposed at an approximately central position of the distal end section 15. The first illumination window 229 is disposed near the first imaging section 228.

The middle step section 226 includes a flat face 226a approximately parallel to the flat face 225a of the projecting step section 225. A first lens 261a that is an observation lens of a second imaging section (a second observing section) 230 for ordinary observation where a non-contacting state with an object is maintained and two (second and third) illumination windows (illumination sections) 231 and 232 are disposed on the flat face 226a of the middle step section 226. Here, the second and third illumination windows 231 and 232 are disposed on both sides of the second imaging section 230. Further, an inclined face 225b with an inclined angle, for example, about 45° is formed on a wall section between the middle step section 226 and the projecting step section 225.

Incidentally, a step between the flat face 225a of the projecting step section 225 and the flat face 226a of the middle step section 226 is set to a height which can prevent the projecting step section 225 from entering in field of view of the second imaging section 230, for example, about 0.7 mm.

The lower step section 227 comprises a flat face 227a approximately parallel to the flat face 225a of the projecting step section 225. A distal end opening section 233a of an endo-therapy device insertion channel 233 disposed inside the insertion section 11 and an air-supplying/water-supplying nozzle 234 are disposed on the flat face 227a of the lower step section 227.

Further, an inclined face 226b with an inclined angle of, for example, about 45° and a fluid guide face 226c with an inclined angle is smaller than that of the inclined face 226b are formed on a wall section between the lower step section 227 and the middle step section 226. The fluid guide face 226c is disposed between the air-supplying/water-supplying nozzle 234 of the lower step section 227 and the second imaging section 230 of the middle step section 226. The fluid guide face 226c is formed by a gentle inclined face with an inclined angle of, for example, about 18°.

A distal end section of the air-supplying/water-supplying nozzle 234 is disposed toward the first lens 261a that is the observation lens of the second imaging section 230. Further, a jetting port 234a of the distal end opening section of the air-supplying/water-supplying nozzle 234 is disposed so as to face the fluid guide face 226c.

In the present embodiment, a pipe conduit opening section 30a of the pipe conduit (a forward water-supplying channel) 30 for fluid supply such as cleaning water for forward water-supply or stain solution, which has been shown in the first embodiment is disposed on the flat face 225a of the projecting step section 225. Further, a recessed guide groove (a flow passage section) 36 for guiding fluid caused to flow from the opening section 30a of the forward water-supplying pipe conduit 30 in a direction of an observation window of an observing section 28 is provided on the flat face 225a of the projecting step section 225.

With the configuration described above, even when stain solution is sprayed from the opening section 30a of the distal end cover 24 of the insertion section 11 in a state that the flat face 225a of the projecting step section 225 has been mainly pushed against a surface of body tissue H, stain solution caused to flow from the opening section 30a of the distal end cover 24 is guided in a direction of the first lens 241a which is the observation lens of the first imaging section 228 by the guide groove 36. Therefore, since stain solution such as methylene blue is surely sprayed in the gap between the first lens 241a that is the observation lens of the observing section 28 and a surface of body tissue H in a state that the first lens 241a which is the observation lens of the first imaging section 228 has been pushed against the surface of the body tissue H at the object contacting observation time with a high magnification ratio where cell tissue to be observed or the like is observed at a high magnification ratio, a site interested is stained so that a contour of a cell can be observed sharply.

Further, the present invention is not limited to the above embodiments, and it can be implemented with variously changed or modified aspects without departing from the gist of the present invention.

Next, other characteristic technical matters of the present invention are as follows:
Note
   (Additional Item 1) An endoscope where an endoscope distal end face is brought into contact with or is caused to come close to an object, comprising: an observation window for contacting observation disposed on the endoscope distal end face; a pipe conduit opening section disposed at the endoscope distal end face; and a flow passage section extending from the opening section to the observation window.
   (Additional Item 2) The endoscope according to Additional Item 1, wherein the flow passage section is a groove.
   (Additional Item 3) The endoscope according to Additional Item 1, wherein the pipe conduit and an axis of the endoscope in a longitudinal direction thereof are approximately parallel to each other.
   (Additional Item 4) An endoscope where an endoscope distal end face is brought into contact with an object, comprising: an observation window for contacting observation disposed on the endoscope distal end face; a pipe conduit opening section disposed on the endoscope distal end face; a least one projecting section disposed on a section of the endoscope distal end face positioned near the opening section; and a flow passage section extending from the opening section to the observation window.
   (Additional Item 5) The endoscope according to Additional Item 4, wherein the flow passage section is a groove.
   (Additional Item 6) The endoscope according to Additional Item 4, wherein the projecting section is a nozzle for cleaning the observation window.
   (Additional Item 7) The endoscope according to Additional Item 4, wherein the flow passage section comprises a pair of projecting walls provided on the endoscope distal end face so as to extend from the opening section toward the observation window.
   (Additional Item 8) The endoscope according to Additional Item 4, wherein each of the projecting walls has a height size increasing from the opening section toward the observation window.
   (Additional Item 9) The endoscope according to Additional Item 4, further comprising a fluid feeding pipe conduit including a base pipe section running substantially in conformity with an axis of an endoscope insertion section and an inclined pipe section extending from the base pipe section in a distal end oblique direction, wherein the opening section is provided as an opening of the inclined pipe section.
   (Additional Item 10) The endoscope according to Additional Item 4, wherein the projecting section is formed in a wedge shape, and a higher side of the projecting section is positioned on a lens face side.
   (Effect of Additional Item 1) Though a subject (stomach or the like) secreting much mucus needs to be stained after the mucus has been removed, when the endoscope distal end is brought into contact with a mucosa, secretion of mucus is reduced so that staining can be performed more stably. When the endoscope distal end is not brought into contact with a mucosa, mucus removal conducted by utilizing water-supply or the like in the same manner as the ordinary forward water-supplying or spraying of stain solution is possible. Even in the state that the distal end section has been brought into contact with a mucosa, stain solution can be caused to flow to the contacting observation window via the groove section of the opening section, so that staining to a cell observation site can be performed easily.
   (Effect of Additional Item 4) In addition to the effect of Additional Item 1, by providing the projecting section near the opening section, the flow passage section can be secured more stably even in a contacting state with a living body, so that staining can be performed more stably.

### [Industrial Applicability]

The present invention is effective in a technical field using an endoscope provided with an observation optical system of an object contacting type where the endoscope is inserted into a body cavity and an object is observed in a state that a distal end section of an objective optical system has been brought into contact with the object, and a technical field manufacturing the endoscope.

## Claims

1. An endoscope that includes an observing section (28, 228) that observes a body to be examined in a contacting state with the body to be examined or a proximal state to the body to be examined on a distal end face (25a, 225a) of an insertion section (11) to be inserted into the body to be examined, comprising
a pipe conduit opening section (30a) for fluid supply that supplies fluid to the distal end face (25a, 225a); and a flow passage section (36) that guides fluid caused to flow from the pipe conduit opening section (30a) in a direction of an observation window (118b1, 241a) of the observing section (28, 228).
**characterized in that**
the flow passage section (36) is a guide groove (36) formed in a recessed shape on the distal end face (25a, 225a).

2. The endoscope according to claim 1,
**characterized in that**
the flow passage section (36) is a chamfered section provided on a ridge line of the pipe conduit opening section (30a) and a chamfer amount of the chamfered section is large only in a direction of the observation window (118b1, 241a).

3. The endoscope according to claim 1,
**characterized in that**
the insertion section (11) comprises a pipe conduit (30) for fluid supply communicating with the pipe conduit opening section (30a) and the pipe conduit (30) is disposed approximately parallel to an axial direction of the center axis of the insertion section (11).

4. The endoscope according to claim 1,
**characterized in that**
the flow passage section (36) has a projecting section (34, 41a, 41b) which is caused to project forward at a peripheral site of the pipe conduit opening section (30a) on the distal end face (25a, 225a) and forms a gap (S) for guiding fluid caused to flow from the pipe conduit opening section (30a) in the direction of the observation window (118b1, 241a) between the peripheral edge site of the pipe conduit .opening section (30a) and the body to be examined when the distal end face (25a, 225a) is brought into contact with the body to be examined.

5. The endoscope according to claim 1,
**characterized by** comprising
a projecting section (34, 41a, 41b) which is caused to project forward at a peripheral site of the pipe conduit opening section (30a) on the distal end face (25a, 225a), and
the projecting section (34, 41a, 41b) is disposed at a peripheral edge site of the guide groove (36).

6. The endoscope according to claim 5,
**characterized in that**
the projecting section (41a, 41b) has a pair of projecting pieces (41a, 41b) disposed on both sides of the guide groove (36).

7. The endoscope according to claim 4,
**characterized in that**
the projecting section (34) is a nozzle (34) for jetting cleaning water for cleaning the observation window (28).

8. The endoscope according to claim 1,
**characterized in that**
the pipe conduit opening section (30a) is caused to communicate with stain solution supplying means (9) for supplying stain solution staining the body to be examined.

9. The endoscope according to claim 1,
**characterized in that**
the pipe conduit opening section (30a) is connected to the stain solution supplying means (9) for supplying stain solution staining the body to be examined and cleaning water supplying means (7) for supplying cleaning water, respectively, and
an operation section (12) of the endoscope has control means (109b) for conducting selective switching between the stain solution supplying means (9) and the cleaning water supplying means (7) to drive the switched means.

## Patentansprüche

1. Endoskop mit einem Beobachtungsabschnitt (28, 228), der einen zu untersuchenden Körper in einem Kontaktzustand mit dem zu untersuchenden Körper oder in einem dem zu untersuchenden Körper nahen Zustand an einer distalen Endfläche (25a, 225a) eines in den zu untersuchenden Körper einzuführenden Einführungsabschnitts (11) beobachtet, wobei das Endoskop umfasst:
einen Leitungselementöffnungsabschnitt (30a) zum Zuführen von Flüssigkeit, der der distalen Endfläche (25a, 225a) Flüssigkeit zuführt; und einen Strömungskanalabschnitt (36) zum Führen von Flüssigkeit, die dazu veranlasst wird von dem Leitungselementöffnungsabschnitt (30a) in Richtung eines Beobachtungsfensters (118b1, 241a) des Beobachtungsabschnitts (28, 228) zu fließen,
**dadurch gekennzeichnet, dass**
der Strömungskanalabschnitt (36) eine in Form einer Aussparung in der distalen Endfläche (25a, 225a) vorgesehene Führungsnut (36) ist.

2. Endoskop nach Anspruch 1,
**dadurch gekennzeichnet, dass**
der Strömungskanalabschnitt (36) ein an einer Kantenlinie des Leitungselementöffnungsabschnitts (30a) vorgesehener abgeschrägter Abschnitt ist, und dass der abgeschrägte Abschnitt nur in Richtung des Beobachtungsfensters (118b1, 241a) einen hohen Abschrägungsgrad aufweist.

3. Endoskop nach Anspruch 1,
**dadurch gekennzeichnet, dass**
der Einführungsabschnitt (11) ein Leitungselement (30) zum Zuführen von Flüssigkeit aufweist, das mit dem Leitungselementöffnungsabschnitt (30a) in Verbindung steht, und dass das Leitungselement (30) sich in etwa parallel zu einer axialen Richtung der Mittenachse des Einführungsabschnitts (11) erstreckt.

4. Endoskop nach Anspruch 1,
**dadurch gekennzeichnet, dass**
der Strömungskanalabschnitt (36) einen vorspringenden Abschnitt (34, 41a, 41b) aufweist, der auf der distalen Endfläche (25a, 225a) nach vorne vorstehend an einer peripheren Stelle des Leitungselementöffnungsabschnitts (30a) ausgebildet ist und einen Spalt (S) bildet, der Flüssigkeit, die dazu veranlasst wird von dem Leitungselementöffnungsabschnitt (30a) in Richtung des Beobachtungsfensters (118b1, 241a) zu fließen, zwischen die periphere Randstelle des Leitungselementöffnungsabschnitts (30a) und den zu untersuchenden Körper leitet, wenn die distale Endfläche (25a, 225a) mit dem zu untersuchenden Körper in Kontakt gebracht wird.

5. Endoskop nach Anspruch 1,
**dadurch gekennzeichnet, dass**
es einen vorspringenden Abschnitt (34, 41a, 41b) aufweist, der auf der distalen Endfläche (25a, 225a) nach vorne vorstehend an einer peripheren Stelle des Leitungselementöffnungsabschnitts (30a) ausgebildet ist, und
dass der vorspringende Abschnitt (34, 41a, 41b) an einer peripheren Randstelle der Führungsnut (36) angeordnet ist.

6. Endoskop nach Anspruch 5,
**dadurch gekennzeichnet, dass**
der vorspringende Abschnitt (41a, 41b) ein Paar vorstehender Teile (41a, 41b) umfasst, die auf den beiden Seiten der Führungsnut (36) angeordnet sind.

7. Endoskop nach Anspruch 4,
**dadurch gekennzeichnet, dass**
der vorspringenden Abschnitt (34) eine Düse (34) ist, zum Ausstoßen von das Beobachtungsfenster (28) reinigendem Reinigungswasser.

8. Endoskop nach Anspruch 1,
**dadurch gekennzeichnet, dass**
der Leitungselementöffnungsabschnitt (30a) in Verbindung mit einer Färbelösungzuführeinrichtung (9) gebracht wird, um dem zu untersuchenden Körper eine Färbelösung zuzuführen.

9. Endoskop nach Anspruch 1,
**dadurch gekennzeichnet, dass**
der Leitungselementöffnungsabschnitt (30a) mit der Färbelösungzuführeinrichtung (9) zum Zuführen einer den zu untersuchenden Körper färbenden Färbelösung bzw. mit der Reinigungswasserzuführeinrichtung (7) zum Zuführen von reinigendem Wasser verbunden ist, und
dass ein Bedienungsabschnitt (12) des Endoskops eine Steuerungseinrichtung (109b) umfasst, zum wahlweisen Umschalten zwischen der Färbelösungzuführeinrichtung (9) und der Reinigungswasserzuführeinrichtung (7) zum Betreiben der jeweils geschalteten Einrichtung.

## Revendications

1. Endoscope qui comprend une partie d'observation (28, 228) qui observe un corps destiné à être examiné dans un état de contact avec le corps destiné à être examiné ou dans un état proximal du corps destiné à être examiné sur une face d'extrémité distale (25a, 225a) d'une section d'insertion (11) destinée à être insérée à l'intérieur du corps destiné à être examiné, comprenant
- une section d'ouverture d'un conduit de tuyau (30a) pour l'alimentation en fluide qui alimente en fluide la face d'extrémité distale (25a, 225a) ; et une section de passage d'écoulement (36) qui guide le fluide entraîné à s'écouler à partir de la section d'ouverture du conduit de tuyau (30a) dans une direction d'une fenêtre d'observation (118b1, 241a) de la section d'observation (28, 228), **caractérisé en ce que**
- la section de passage de flux (36) est une gorge de guidage (36) formée dans une forme en renfoncement de la face d'extrémité distale (25a, 225a).

2. Endoscope selon la revendication 1, **caractérisé en ce que** la section de passage d'écoulement (36) est une section chanfreinée réalisée sur une ligne d'arête de la section d'ouverture du conduit de tuyau (30a) et une quantité de chanfrein de la section chanfreinée est large seulement dans une direction de la fenêtre d'observation (118ba, 241a).

3. Endoscope selon la revendication 1, **caractérisé en ce que** la section d'insertion (11) comprend un conduit de tuyau (30) pour l'alimentation en fluide communiquant avec la section de passage de conduit de tuyau (30a) et le conduit de tuyau (30) est agencé sensiblement parallèlement à une direction axiale de l'axe centrale de la section d'insertion (11).

4. Endoscope selon la revendication 1, **caractérisé en ce que** la section de passage d'écoulement a une section en saillie (34, 41a, 41b) laquelle est induite à se mettre en saillie vers l'avant au niveau d'un site périphérique de la section d'ouverture du conduit de tuyau (30a) sur la face d'extrémité distale (25a, 225a) et forme un espacement (S) pour guider le fluide entraîné à s'écouler à partir de la section d'ouverture du conduit de tuyau (30a) dans la direction de la fenêtre d'observation (118b1, 241a) entre le site du bord périphérique de la section d'ouverture du conduit de tuyau (30a) et le corps destiné à être examiné lorsque la face d'extrémité distale (25a, 225a) est amenée en contact avec le corps destiné à être examiné.

5. Endoscope selon la revendication 1, **caractérisé en ce qu'**il comprend une section en saillie (34, 41a, 41b) laquelle est induite à se mettre en saillie vers l'avant au niveau d'un site périphérique de la section d'ouverture du conduit de tuyau (30a) sur la face d'extrémité distale (25a, 225a), et la section en saillie (34, 41a, 41b) est agencée au niveau d'un site du bord périphérique de la gorge de guidage (36).

6. Endoscope selon la revendication 5, **caractérisé en ce que** la section en saillie (41a, 41b) a une paire de pièces en saillie (41a, 41b) agencées des deux côtés de la gorge de guidage (36).

7. Endoscope selon la revendication 4, **caractérisé en ce que** la section en saillie (34) est une buse pour projeter de l'eau de nettoyage pour nettoyer la fenêtre d'observation (28).

8. Endoscope selon la revendication 1, **caractérisé en ce que** la section d'ouverture du conduit de tuyau (30a) est induite à communiquer avec des moyens d'alimentation en solution colorante (9) pour alimenter en solution colorante colorant le corps destiné à être examiné.

9. Endoscope selon la revendication 1, **caractérisé en ce que** la section d'ouverture de conduit de tuyau (30a) est reliée aux moyens d'alimentation en solution colorante (9) pour alimenter en solution colorante colorant le corps destiné à être examiné et des moyens d'alimentation en eau de nettoyage (7) pour alimenter en eau de nettoyage, respectivement, et
une section d'actionnement (12) de l'endoscope a des moyens de commande (109b) pour effectuer une commutation sélective entre les moyens d'alimentation en solution colorante (9) et les moyens d'alimentation en eau de nettoyage (7) pour entraîner les moyens de commutation.
